# EUROPÄISCHE PATENTSCHRIFT

(11) **EP 3 527 973 B1**
(45) Veröffentlichungstag und Bekanntmachung des Hinweises auf die Patenterteilung: **13.10.2021**
(21) Anmeldenummer: 19158097.6
(22) Anmeldetag: 19.02.2019
(51) Int. Cl.: G01N 21/85, F23G 5/50, G01N 21/3563, G01N 33/22

(54) **VERFAHREN ZUR BESTIMMUNG DER QUALITÄT VON ERSATZBRENNSTOFFPARTIKELN**
METHOD FOR DETERMINING THE QUALITY OF REFUSE-DERIVED FUEL PARTICLES
PROCÉDÉ DE DÉTERMINATION DE LA QUALITÉ DE PARTICULES DE COMBUSTIBLE DÉRIVÉ DE DÉCHETS

(30) Priorität: 20.02.2018 AT 5003118 U
(43) Veröffentlichungstag der Anmeldung: 21.08.2019
(73) Patentinhaber: EVK DI Kerschhaggl GmbH, 8074 Raaba (AT)
(72) Erfinder: Kerschhaggl, Matthias, 8010 Graz (AT); Kerschhaggl, Peter, 8074 Raaba (AT)
(74) Vertreter: Margotti, Herwig Franz

(56) Entgegenhaltungen:
- WO-A1-2017/009156
- WO-A1-2017/009158
- DE-A1-102010 031 528
- JP-A- 2001 013 069

## Beschreibung

Die Erfindung betrifft ein Verfahren zur Bestimmung der Qualität von Ersatzbrennstoffpartikeln mit den Merkmalen des Oberbegriffs von Anspruch 1.

Die Erfindung betrifft des Weiteren eine Vorrichtung zur Bestimmung der Qualität von Ersatzbrennstoffpartikeln mit einer Lichtquelle, einem Fotosensor und einer mit dem Fotosensor verbundenen Auswerteeinheit.

Der Nachweis verschiedener Inhaltsstoffe in einzelnen Partikeln eines Schüttguts ist von Interesse, um beispielsweise eine Unterscheidung der Partikel anhand ihrer Materialzusammensetzung zu treffen. Dies ist insbesondere in Sortier- und/oder Förderanlagen von Vorteil, um in einem Materialstrom aus Partikeln verschiedener Zusammensetzung Partikel eines bestimmten Materials oder mit bestimmten Eigenschaften herauszufiltern oder zu detektieren.

Eine Möglichkeit hierfür ist eine Untersuchung der Partikel des Schüttguts durch Reflektions- oder Absorptionsspektrometrie mithilfe von Fotosensoren. Dies ist eine gängige Methode in vielen Industriebereichen. Hierbei wir das Schüttgut mit Licht, beispielsweise in einem bestimmten Wellenlängenbereich, bestrahlt und ein Absorptionsspektrum beziehungsweise Reflexionsspektrum mittels des Fotosensors erfasst und analysiert. Eine Ausführungsform eines solchen Verfahrens und einer solchen Vorrichtung auf dem Gebiet der Lebensmittelindustrie wird beispielsweise in der Veröffentlichung US 2013/0278919 A1 beschrieben. Bei diesem bekannten Verfahren werden Samen einzeln spektroskopisch untersucht, indem sie mit einer Lichtquelle bestrahlt werden. Anschließend wird ein Absorptions- oder Reflektionsspektrum von einem Fotosensor aufgenommen. Eine Rechnereinheit analysiert daraufhin das Absorptions- oder Reflektionsspektrum jedes Samens in einem Bereich von Interesse und errechnet anhand einer Kalibrierkurve den Gehalt eines bestimmten Inhaltsstoffes des Samens.

Weitere Anlagen zur Sortierung und Analyse von Lebensmitteln sind in der EP 672 468 B1 oder der EP 1 332 353 B1 beschrieben. Überwiegend erfolgt dabei eine Auflicht-Detektion und Sortierung von geförderten Objekten, bei der die geförderten Objekte vollautomatisch inspiziert werden, indem Licht auf sie eingestrahlt wird und das reflektierte Licht von einer Kamera aufgefangen und analysiert wird. Das Licht ist entweder breitbandiges oder gefiltertes Weißlicht, aber auch als LED- oder Laserlicht mit bestimmten für die jeweilige Aufgabenstellung relevanten Wellenlängen ausgeführt. Mit dieser Technologie sind beispielsweise Störstellen an der Objektoberfläche erkennbar, aber auch gewisse Qualitätsunterschiede beim Gutmaterial.

Derartige Technologien werden auch auf dem Gebiet der Qualitätskontrolle in der Abfallwirtschaft eingesetzt. Insbesondere im Bereich der Ersatzbrennstoffe (EBS), auch als refuse derived fuel - RDF bezeichnet, welche in der Regel aus Abfallmaterial gewonnen werden, ist eine Bestimmung der Qualität des Ersatzbrennstoffs von Interesse. Die Qualität von Ersatzbrennstoffen wird durch verschiedene Qualitätsparameter wie unter anderem Feuchtigkeitsgehalt, Chlorgehalt, Heizwert und Brennwert beeinflusst. Lösungen gemäß dem Stand der Technik zur Bestimmung dieser Qualitätsparameter von Ersatzbrennstoffen weisen den Nachteil auf, dass diese nur eine allgemeine und unpräzise Beurteilung dieser Qualitätsparameter bereitstellen. Dies führt in der Regel dazu, dass beispielsweise bei einer nachfolgenden Sortierung von Ersatzbrennstoffmaterial, welches einem Verfahren gemäß dem Stand der Technik unterzogen wurde, ein unnötig hoher Ausschuss erzeugt wird.

Darüber hinaus bewirkt eine ungenaue Bestimmung der Qualität des Ersatzbrennstoffmaterials, das beispielsweise Verbrennungsöfen, welche hiermit beschickt werden, nicht optimal arbeiten.

Die WO 2016/201254 A1 und die WO 2013/102916 A1 offenbaren spektroskopische Verfahren zur Bestimmung einer Eigenschaft eines Rohöls.

Das Dokument US 2013/278919 A1 offenbart ein spektroskopisches Verfahren zur quantitativen Bestimmung von Inhaltsstoffen in einem Samen.

Die US 5,464,981 A offenbart ein spektroskopisches Verfahren zur Unterscheidung von Weintraubenstängeln, Weintraubenblättern, Steinen usw. aus einer Mischung mit Weintrauben.

Die CN 106092981 A offenbart ein spektroskopisches Verfahren zur Unterscheidung von Rohöl und von marinem Rückstandsöl.

Die DE 10 2010 031528 A1 offenbart ein System zur Bestimmung eines Primärenergiegehalts von festem Biomassebrennstoff oder einem Ersatzbrennstoff.

Die WO 2017/009158 A1 offenbart ein Verfahren zur Regelung eines Brennprozesses mit einem Ersatzbrennstoff.

Die WO 2017/009156 A1 offenbart ein Verfahren zur Einhaltung von Emissionsgrenzwerten in einem Brennprozess, wobei wenigstens ein Brennstoff oder wenigstens ein Brennstoffgemisch zum Einsatz kommt.

Der Erfindung liegt somit die Aufgabe zugrunde, ein Verfahren bereitzustellen, welches die Nachteile des Standes der Technik vermeidet.

Erfindungsgemäß wird diese Aufgabenstellung durch ein Verfahren zur Bestimmung der Qualität von Ersatzbrennstoffpartikeln mit den Merkmalen des Anspruchs 1 gelöst.

In dem erfindungsgemäßen Verfahren werden Ersatzbrennstoffpartikel mit zumindest einer Lichtquelle bestrahlt. Die Ersatzbrennstoffpartikel können hierbei entweder einzeln vorliegen, oder beispielsweise auf einem Förderband oder einer anderen Fördervorrichtung an der Lichtquelle vorbeigeführt werden. Das von den Ersatzbrennstoffpartikeln reflektierte oder transmittierte Licht wird im nächsten Verfahrensschritt auf einen Fotosensor projiziert. Alternativ können in dem erfindungsgemäßen Verfahren auch mehrere Fotosensoren verwendet werden. Der Fotosensor erfasst aus dem reflektierten beziehungsweise transmittierten Licht zumindest ein Lichtspektrum eines Ersatzbrennstoffpartikels und übermittelt dieses an eine Auswerteeinheit. Die Auswerteeinheit vergleicht das erfasste Lichtspektrum mit Referenzlichtspektren von Referenz-Ersatzbrennstoffpartikeln aus bekannten Materialien, und bestimmt eine Abweichung der Referenzlichtspektren von dem erfassten Lichtspektrum. Dies ermöglicht eine darauffolgende Zuordnung des Materials des Referenz-Ersatzbrennstoffpartikels dessen Referenzlichtspektrums die geringste Abweichung zu dem erfassten Lichtspektrum aufweist, zu dem erfassten Lichtspektrum durch die Auswerteeinheit. Hierbei werden einzelne spektrale Bildelemente (Spectels) in Hinblick auf ihre Materialzugehörigkeit vorklassifiziert. Im nächsten Verfahrensschritt wird ein quantitativer Qualitätsparameter des Ersatzbrennstoffpartikels, dessen Lichtspektrum erfasst wurde, indem das erfasste Lichtspektrum durch Anwendung einer materialspezifischen, durch ein Regressionsverfahren ermittelten, Korrelationsfunktion, von der Auswerteeinheit ausgewertet wird. Die Korrelationsfunktion korreliert Lichtspektrendaten von Ersatzbrennstoffpartikeln mit referenzanalytisch bestimmten quantitativen Qualitätsparametern von Ersatzbrennstoffen. Diese quantitativen Qualitätsparameter wurden im Vorfeld laboranalytisch, beispielsweise auf nasschemischem Weg, bestimmt und mit Lichtspektrendaten unter Anwendung eines Regressionsverfahrens korreliert. Hierdurch wird der Vorteil erreicht, dass mittels einer schnellen und für einen Anwender einfach durchzuführenden lichtspektrometrischen Analyse von Ersatzbrennstoffpartikeln ein quantitativer Wert eines Qualitätsparameters von Ersatzbrennstoffen bestimmt werden kann.

In einer bevorzugten Ausführungsform der Erfindung korreliert die Korrelationsfunktion Lichtsprektrendaten von Ersatzbrennstoffen mit quantitativen Qualitätsparametern ausgewählt aus Feuchtigkeitsgehalt, Chlorgehalt, Heizwert und Brennwert. Hierdurch wird der Vorteil erreicht, dass verschiedene Parameter quantitativ bestimmt werden und eine spezifische Selektion der Ersatzbrennstoffpartikel nach einem oder einer Kombination von Parametern ermöglicht wird.

Es ist bevorzugt die Korrelationsfunktion durch ein Regressionsverfahren aus dem Bereich des statistisch-maschinellen Lernens zu ermitteln. Insbesondere kommen hierbei Regressionsverfahren ausgewählt aus Partial Least Squares Regression, Hauptkomponentenregression, multiple lineare Regression, Support Vektor Regression, Neuronal Network Regression und Deep Learning Methoden zur Anwendung. Hierdurch wird der Vorteil erreicht, dass z.B. die Varianz oder eine andere informationsrelevante statistische Größe aus einem Referenzdatensatz mit jener im Datensatz der erfassten Lichtspektren für zukünftige, rein auf den neu erfassten Lichtspektren basierende Vorhersagen in Zusammenhang gebracht werden kann.

Die Ersatzbrennstoffpartikel werden gemäß der bevorzugten Ausführungsform des erfindungsgemäßen Erfahrens von der Lichtquelle mit Licht in einem Wellenlängenbereich von 300-2500 nm oder einem Teilbereich davon bestrahlt. Die Auswahl von Wellenlängen des Nahinfrarotbereichs bietet den Vorteil, dass ein sehr aussagekräftiger Informationsgehalt durch die Reflektions-und/oder Absorptionsspektren generiert wird. In einer Ausführungsform der Erfindung werden die die Ersatzbrennstoffpartikel von der Lichtquelle mit Strahlung mit einem diskreten Spektralverlauf bestrahlt.

Die Aufgabenstellung der Erfindung wird zudem durch eine Vorrichtung zur Bestimmung der Qualität von Ersatzbrennstoffpartikeln mit einer Lichtquelle, einem Fotosensor und einer mit dem Fotosensor verbundenen Auswerteeinheit gelöst, wobei die Vorrichtung dazu ausgebildet ist, das erfindungsgemäße Verfahren abzuarbeiten.

In der bevorzugten Ausführungsform der erfindungsgemäßen Vorrichtung umfasst die Vorrichtung eine mit der Auswerteeinheit verbundene Ausgabeeinheit, wobei die Ausgabeeinheit dazu ausgebildet ist, zumindest eines aus dem zumindest einen bestimmten quantitativen Qualitätsparameter, dem zugeordneten Material und dem erfassten Lichtspektrum auszugeben. Hierdurch wird der Vorteil erreicht, dass einem Benutzer der erfindungsgemäßen Vorrichtung ermöglicht wird, die von der Vorrichtung ermittelten Informationen unmittelbar abzulesen.

Des Weiteren kann die Vorrichtung eine Fördereinheit umfassen, wobei die Fördereinheit dazu ausgebildet ist, Ersatzbrennstoffpartikel an der Lichtquelle vorbeizuführen. Die Verbindung der Vorrichtung mit einer Fördereinheit ermöglicht vorteilhafterweise, die erfindungsgemäße Vorrichtung in einer automatisierten Prozessanlage, wie beispielsweise einem automatisch beschickten Verbrennungsofen, einzusetzen.

Zudem ist in der bevorzugten Ausführungsform die Auswerteeinheit mit einer Signaleinheit verbunden, wobei die Signaleinheit dazu ausgebildet ist, bei Überschreiten und/oder Unterschreiten eines Alarmwertes durch den bestimmten quantitativen Qualitätsparameter ein Signal zu erzeugen. Hierdurch wird der Vorteil erreicht, dass ein Benutzer der einfindungsgemäßen Vorrichtung beispielsweise auf unvorteilhafte Eigenschaften des mit der Vorrichtung überprüften Ersatzbrennstoffmaterials aufmerksam gemacht wird. So kann der Benutzer z.B. bei Unterschreitung des gewünschten Heizwertes bei der Beschickung eines Drehrohrofens mittels Fluff zusätzlich mit Koks zuheizen.

Darüber hinaus ist in der bevorzugten Ausführungsform der Vorrichtung die Fördereinheit mit der Auswerteeinheit verbunden, wobei die Fördereinheit dazu ausgebildet ist, bei Überschreiten und/oder Unterschreiten eines Alarmwertes durch den bestimmten quantitativen Qualitätsparameter anzuhalten. Hierdurch wird der Vorteil erreicht, dass bei Verwendung der erfindungsgemäßen Vorrichtung in einer automatisierten Verbrennungsanlage negative Auswirkungen auf den Verbrennungsprozess, beispielsweise durch qualitativ minderwertigen Ersatzbrennstoff, automatisch verhindert wird.

Vorteilhafte Ausgestaltungen des erfindungsgemäßen Verfahrens und der Vorrichtung, sowie alternativer Ausführungsvarianten werden in weiterer Folge anhand der Figuren näher erklärt.
Figur 1 zeigt eine Vorrichtung zur Bestimmung der Qualität von Ersatzbrennstoffpartikeln in einer schematischen Darstellung.
Figur 2 zeigt die Vorrichtung zur Bestimmung der Qualität von Ersatzbrennstoffpartikeln mit einer gesteuerten Fördereinheit.

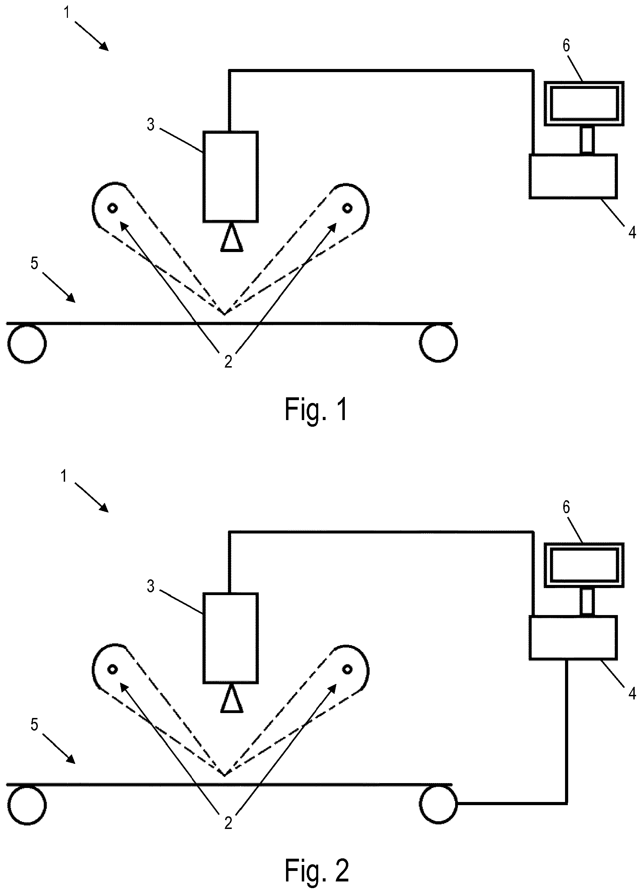

Figur 1 zeigt eine erfindungsgemäße Vorrichtung 1 zur Bestimmung der Qualität von Ersatzbrennstoffpartikeln, welche ein erfindungsgemäßes Verfahren zur Bestimmung der Qualität von Ersatzbrennstoffpartikeln abarbeitet. Die erfindungsgemäße Vorrichtung 1 umfasst eine Lichtquelle 2, einen Fotosensor 3 und eine mit dem Fotosensor 3 verbundene Auswerteeinheit 4. Die in Figur 1 dargestellte Vorrichtung 1 umfasst darüber hinaus eine Fördereinheit 5 und eine mit der Auswerteeinheit 3 verbundene Ausgabeeinheit 6. Der Fotosensor 3 kann beispielsweise in Form einer Hyperspektralkamera oder einer Multispektralkamera vorliegen, und die Auswerteeinheit 4 kann beispielsweise durch eine Rechnereinheit, wie beispielsweise einen PC, mit einer CPU, FPGA und/oder GPU, die auch im Sinne eines eingebetteten Systems in der Kameraeinheit selbst oder aber in einem Schaltschrank verbaut sein kann, gebildet werden. Hierdurch wird der Vorteil erreicht, dass mittels ortsaufgelöster Spektroskopie einzelne Brennstoffpartikel im Materialstrom charakterisiert werden können. Die Fördereinheit kann beispielsweise ein Förderband, ein Schneckenförderer oder Ähnliches sein. Die Lichtquelle 2 bestrahlt Ersatzbrennstoffpartikel, welche in Figur 1 nicht dargestellt sind, und in der in Figur 1 dargestellten Vorrichtung 1 von der Fördereinheit 5 an der Lichtquelle 2 in einer Förderrichtung vorbeigeführt werden. Die in der bevorzugten Ausführungsform der Vorrichtung 1 vorhandene Fördereinheit 5 bietet den Vorteil, dass die erfindungsgemäße Vorrichtung 1 hierdurch als Teil einer automatisierten Prozessanlage oder Produktionsanlage, wie einem automatisch beschickten Verbrennungsofen, eingesetzt werden kann. Zudem umfasst die in Figur 1 dargestellte Lichtquelle 2 zwei Beleuchtungseinheiten, wie beispielsweise LED Elemente oder Halogenleuchten mit Reflektoren zur Fokussierung des erzeugten Lichts auf die Ersatzbrennstoffpartikel. Das von den Ersatzbrennstoffpartikeln reflektierte Licht wird auf den Fotosensor 3 projiziert. Alternativ kann auch das transmittierte Licht auf den Fotosensor 3 projiziert werden, wobei in dieser Ausführungsvariante der Vorrichtung 1 der Fotosensor 3 auf der, in Figur 1 gegenüberliegenden, Seite der Fördereinheit 5 angeordnet ist. Es sind auch Kombinationen dieser beiden Varianten unter Erfassung sowohl des transmittierten als auch des reflektierten Lichts realisierbar. Der Fotosensor 3 erfasst zumindest ein Lichtspektrum eines Ersatzbrennstoffpartikels aus dem reflektierten und/oder transmittierten Licht und übermittelt das erfasste Lichtspektrum an die mit dem Fotosensor 3 verbundene Auswerteeinheit 4. Im nächsten Schritt des erfindungsgemäßen Verfahrens vergleicht die Auswerteeinheit 4 das erfasste Lichtspektrum zumindest bereichsweise mit Referenzlichtspektren von Referenz-Ersatzbrennstoffpartikeln aus bekannten Materialien, wie beispielsweise Polypropylen PP, Polyvinylchlorid PVC, Polystyrol PS, Polyethylenterephthalat PET oder Holz. Hierbei bestimmt die Auswerteeinheit 4 eine Abweichung der Referenzlichtspektren von dem erfassten Lichtspektrum und ordnet das Material des Referenz-Ersatzbrennstoffpartikels dessen Referenzlichtspektrums die geringste Abweichung von dem erfassten Lichtspektrum aufweist, zu dem erfassten Lichtspektrum zu.

Die Abweichung kann beispielsweise eine Abweichung in Bezug auf einen Mittelwert des Lichtspektrums darstellen, oder auf die Höhe einzelner Peaks des Lichtspektrums bezogen sein. Hierbei_kommen beispielsweise diverse Datencluster-Analyse-Methoden wie Baumdiagramme, k-means clustering, Malahanobis- Abstand und diverse andere Metriken oder auch Endmember-Bestimmungsalgorithmen wie spectral unmixing, Pixel Purity Index (PPI), N-FINDER und Automated Morphological Endmember Extraction (AMEE) zur Anwendung. Hierdurch wird vorteilhafterweise ermöglicht festzustellen, aus welchem Material Ersatzbrennstoffpartikel bestehen. Im nächsten Verfahrensschritt bestimmt die Auswerteeinheit 4 einen quantitativen Qualitätsparameter des erfassten Lichtspektrums.

Hierbei wertet die Auswerteeinheit 4 das erfasste Lichtspektrum zumindest bereichsweise mittels einer materialspezifischen Korrelationsfunktion aus, welche Lichstpektrendaten von Ersatzbrennstoffpartikeln mit referenzanalytisch bestimmten quantitativen Qualitätsparametern von Ersatzbrennstoffen korreliert. Diese quantitativen Qualitätsparameter wurden im Vorfeld durch labortechnische Referenzanalytik, beispielsweise auf nasschemischem Weg, beziehungsweise kalorimetrisch bestimmt und mit den Lichtspektrendaten unter Anwendung eines Regressionsverfahrens korreliert. Zur Erstellung der Korrelationsfunktion werden beispielsweise Nahinfrarot Spektren einer möglichst großen Anzahl von Proben von Ersatzbrennstoffpartikeln erstellt, normiert, und anschließend mit labortechnisch bestimmten Referenzwerten für zumindest einen der quantitativen Qualitätsparameter mittels multivarianter Datenanalyse korreliert. Hierzu werden die quantitativen Qualitätsparameter der Proben nach Erstellung der Nahinfrarot Spektren mittels labortechnischer Analytik bestimmt. Die Anzahl an Latenzvariablen wird hierbei bevorzugt stets so gewählt, dass die pro Variable erklärte Varianz zumindest im Prozentbereich liegt, um Korrelationen mit reinem Rauschen zu unterdrücken. Hierdurch wird der Vorteil erreicht, dass durch das erfindungsgemäße Verfahren und die erfindungsgemäße Vorrichtung 1 nicht nur eine Bestimmung des Materials der Ersatzbrennstoffpartikel bereitgestellt wird, sondern darüber hinaus eine Beurteilung der Qualität der Ersatzbrennstoffpartikel anhand von quantitativen Qualitätsparametern ermöglicht wird.

Gemäß der bevorzugten Ausführungsform des erfindungsgemäßen Verfahrens korreliert die Korrelationsfunktion Lichtspektrendaten von Ersatzbrennstoffen mit quantitativen Qualitätsparametern, wie beispielsweise Feuchtigkeitsgehalt, Chlorgehalt, Heizwert und Brennwert. Das erfindungsgemäße Verfahren erlaubt auch die simultane Bestimmung von einem oder mehreren dieser Qualitätsparameter. Hierdurch wird der Vorteil erreicht, dass verschiedene Parameter quantitativ bestimmt werden und eine spezifische Selektion der Ersatzbrennstoffpartikel nach einem oder einer Kombination von Parametern ermöglicht wird.

Gemäß der bevorzugten Ausführungsform wird die Korrelationsfunktion durch ein Regressionsverfahren aus dem Bereich des statistisch-maschinellen Lernens zu ermittelt. Insbesondere kommen hierbei Regressionsverfahren ausgewählt aus Partial Least Squares Regression, Hauptkomponentenregression, multiple lineare Regression Support Vektor Regression, Neuronal Network Regression und Deep Learning Methoden zur Anwendung. Hierdurch wir der Vorteil erreicht, dass z.B. die Varianz oder eine andere informationsrelevante statistische Größe aus einem Referenzdatensatz mit jener im Datensatz der erfassten Lichtspektren für zukünftige, rein auf den neu erfassten Lichtspektren basierende Vorhersagen in Zusammenhang gebracht werden kann.

Die Ersatzbrennstoffpartikel werden in der bevorzugten Ausführungsform von der Lichtquelle 2 mit Licht in einem Wellenlängenbereich von 300-2500 nm oder einem Teilbereich davon bestrahlt. Insbesondere können die Ersatzbrennstoffpartikel von der Lichtquelle 2 auch mit Strahlung mit einem diskreten Spektralverlauf bestrahlt werden. Hierdurch wird der Vorteil erreicht, dass durch die Auswahl von gezielten Wellenlängen zum Beispiel des Nahinfrarotbereichs, ein möglichst großer Informationsgehalt durch die Reflektions-und/oder Absorptionsspektren generiert wird.

Die mit der Auswerteeinheit 4 verbundene Ausgabeeinheit 6 gibt gemäß der bevorzugten Ausführungsform der erfindungsgemäßen Vorrichtung 1 zumindest einen bestimmten quantitativen Qualitätsparameter, dem zugeordneten Material und/oder dem erfassten Lichtspektrum oder die erfassten Lichtspektren aus.

In bevorzugten Ausführungsform der Vorrichtung 1 ist die Auswerteeinheit 4 der Weiteren mit einer, in Figur 1 nicht dargestellten, Signaleinheit verbunden. Die Signaleinheit erzeugt bei überschreiten und/oder unterschreiten eines Alarmwertes durch den bestimmten quantitativen Qualitätsparameter ein beispielsweise optisches oder akustisches Signal.

Figur 2 zeigt eine Ausführungsvariante der erfindungsgemäßen Vorrichtung 1, bei der die Fördereinheit 5 mit der Auswerteeinheit 4 verbunden ist. Die Fördereinheit 5 hält in dieser Ausführungsvariante der Vorrichtung 1 an, sobald der quantitative Qualitätsparameter einen Alarmwert überschreitet und/oder unterschreitet. Hierdurch wird der Vorteil erreicht, dass die erfindungsgemäßen Vorrichtung 1 in einer automatisierten Anlage eingesetzt werden kann.

Gemäß der bevorzugten Ausführungsform erstellt der Fotosensor 3 eine Hyperspektralaufhahme des Ersatzbrennstoffpartikels. Hierdurch wird der Vorteil erreicht, dass mittels ortsaufgelöster Spektroskopie einzelne Brennstoffpartikel im Materialstrom charakterisiert werden können.

Gemäß einer weiteren Ausführungsvariante der erfindungsgemäßen Vorrichtung 1 ohne Fördereinheit 5 kann die Vorrichtung beispielsweise in einem tragbaren Gehäuse bereitgestellt sein. Hierdurch wird der Vorteil erreicht, dass die Vorrichtung 1 mobil in Form eines Handheld-Analysegeräts einsetzbar ist. So können beispielsweise Eingangs- oder Ausgangskontrollen von Ersatzbrennstoffen in Form z.B. einer Batchanalyse, die auf dem erfindungsgemäßen Verfahren aufbauen durchgeführt werden. Besonders vorteilhaft ist, dass hierdurch beispielsweise Qualitätskriterien bei der Anlieferung oder die Güteklasse in der Produktion anhand von Stichproben mit relevanter Proebenmenge (Batch) überprüft werden können.

## Patentansprüche

1. Verfahren zur Bestimmung der Qualität von Ersatzbrennstoffpartikeln umfassend die Schritte:
a. Bestrahlen von Ersatzbrennstoffpartikeln mit zumindest einer Lichtquelle (2),
b. Projizieren des von den Ersatzbrennstoffpartikeln reflektierten und/oder transmittierten Lichts auf zumindest einen Fotosensor (3),
c. Erfassen zumindest eines Lichtspektrums eines Ersatzbrennstoffpartikels aus dem reflektierten und/oder transmittierten Licht durch den zumindest einen Fotosensor (3) und Übermitteln des erfassten Lichtspektrums an eine mit dem Fotosensor (3) verbundene Auswerteeinheit (4),
d. zumindest bereichsweises Vergleichen des erfassten Lichtspektrums mit Referenzlichtspektren von Referenz-Ersatzbrennstoffpartikeln aus bekannten Materialien, Bestimmen einer Abweichung der Referenzlichtspektren von dem erfassten Lichtspektrum und Zuordnen des Materials des Referenz-Ersatzbrennstoffpartikels, dessen Referenzlichtspektrum, die geringste Abweichung von dem erfassten Lichtspektrum aufweist, zu dem erfassten Lichtspektrum durch die Auswerteeinheit (4),
**gekennzeichnet durch** den Schritt
e. Bestimmen zumindest eines quantitativen Qualitätsparameters des Ersatzbrennstoffpartikels, dessen Lichtspektrum erfasst wurde, durch zumindest bereichsweises Auswerten des erfassten Lichtspektrums mittels einer materialspezifischen, durch ein Regressionsverfahren ermittelten, Korrelationsfunktion zwischen Lichtspektrendaten von Ersatzbrennstoffpartikeln und referenzanalytisch bestimmten quantitativen Qualitätsparametern von Ersatzbrennstoffen durch die Auswerteeinheit (4).

2. Verfahren gemäß Anspruch 1, **dadurch gekennzeichnet, dass** die Korrelationsfunktion Lichtsprektrendaten von Ersatzbrennstoffen mit quantitativen Qualitätsparametern ausgewählt aus Feuchtigkeitsgehalt, Chlorgehalt, Heizwert und Brennwert korreliert.

3. Verfahren gemäß einem der Ansprüche 1 oder 2, **dadurch gekennzeichnet, dass** die Korrelationsfunktion durch ein Regressionsverfahren aus dem Bereich des statistisch-maschinellen Lernens ermittelt wird.

4. Verfahren gemäß Anspruch 3, **dadurch gekennzeichnet, dass** die Korrelationsfunktion durch ein Regressionsverfahren ausgewählt aus Partial Least Squares Regression, Hauptkomponentenregression, multiple lineare Regression, Support Vektor Regression, Neuronal Network Regression und Deep Learning Methoden ermittelt wird.

5. Verfahren gemäß einem der Ansprüche 1 bis 4, **dadurch gekennzeichnet, dass** die Ersatzbrennstoffpartikel von der Lichtquelle (2) mit Licht in einem Wellenlängenbereich von 300-2500 nm oder einem Teilbereich davon bestrahlt werden.

6. Verfahren gemäß einem der Ansprüche 1 bis 4, **dadurch gekennzeichnet, dass** die Ersatzbrennstoffpartikel von der Lichtquelle (2) mit Strahlung mit einem diskreten Spektralverlauf bestrahlt werden.

7. Verfahren gemäß einem der Ansprüche 1 bis 6, **dadurch gekennzeichnet, dass** der Fotosensor (3) eine Hyperspektralaufnahme des Ersatzbrennstoffpartikels erstellt.

8. Vorrichtung (1) zur Bestimmung der Qualität von Ersatzbrennstoffpartikeln mit einer Lichtquelle (2), einem Fotosensor (3) und einer mit dem Fotosensor (3) verbundenen Auswerteeinheit (4), wobei die Lichtquelle (2) dazu ausgebildet ist, Ersatzbrennstoffpartikel zu bestrahlen, und der Fotosensor (3) zur Projektion von, von den Ersatzbrennstoffpartikeln reflektiertem und/oder transmittierten Licht auf den Fotosensor (3) angeordnet ist, und der Fotosensor (3) zum Erfassen zumindest eines Lichtspektrums eines Ersatzbrennstoffpartikels aus dem reflektierten und/oder transmittierten Licht und zum Übermitteln des erfassten Lichtspektrums an die Auswerteeinheit (4) ausgebildet ist, wobei die Auswerteeinheit (4) dazu ausgebildet ist, das erfasste Lichtspektrum zumindest bereichsweise mit Referenzlichtspektren von Referenz-Ersatzbrennstoffpartikeln aus bekannten Materialien zu vergleichen, eine Abweichung der Referenzlichtspektren von dem erfassten Lichtspektrum zu bestimmen, und das Material des Referenz-Ersatzbrennstoffpartikels, dessen Referenzlichtspektrum, die geringste Abweichung von dem erfassten Lichtspektrum aufweist, zu dem erfassten Lichtspektrum zuzuordnen,
**dadurch gekennzeichnet, dass**
die Auswerteeinheit (4) dazu ausgebildet ist, zumindest einen quantitativen Qualitätsparameter des Ersatzbrennstoffpartikels, dessen Lichtspektrum erfasst wurde durch zumindest bereichsweises Auswerten des erfassten Lichtspektrums mittels einer materialspezifischen, durch ein Regressionsverfahren ermittelten, Korrelationsfunktion zwischen Lichtspektrendaten von Ersatzbrennstoffpartikeln und referenzanalytisch bestimmten quantitativen Qualitätsparametern von Ersatzbrennstoffen zu bestimmen.

9. Vorrichtung (1) gemäß Anspruch 8, **dadurch gekennzeichnet, dass** die Vorrichtung (1) eine mit der Auswerteeinheit (4) verbundene Ausgabeeinheit (6) umfasst, wobei die Ausgabeeinheit (6) dazu ausgebildet ist zumindest eines aus dem zumindest einen bestimmten quantitativen Qualitätsparameter, dem zugeordneten Material und dem erfassten Lichtspektrum auszugeben.

10. Vorrichtung (1) gemäß einem der Ansprüche 8 oder 9, **dadurch gekennzeichnet, dass** die Vorrichtung (1) eine Fördereinheit (5) umfasst, wobei die Fördereinheit (5) dazu ausgebildet ist, Ersatzbrennstoffpartikel an der Lichtquelle (2) vorbeizuführen.

11. Vorrichtung (1) gemäß Anspruch 10, **dadurch gekennzeichnet, dass** die Fördereinheit (5) mit der Auswerteeinheit (4) verbunden ist, wobei die Fördereinheit (5) dazu ausgebildet ist, bei Überschreiten und/oder Unterschreiten eines Alarmwertes durch den bestimmten quantitativen Qualitätsparameter anzuhalten.

12. Vorrichtung (1) gemäß einem der Ansprüche 8 bis 11, **dadurch gekennzeichnet, dass** die Auswerteeinheit (4) mit einer Signaleinheit verbunden ist, wobei die Signaleinheit dazu ausgebildet ist, bei Überschreiten und/oder Unterschreiten eines Alarmwertes durch den bestimmten quantitativen Qualitätsparameter ein Signal zu erzeugen.

13. Vorrichtung (1) gemäß einem der Ansprüche 8 bis 12, **dadurch gekennzeichnet, dass** der Fotosensor (3) eine Hyperspektralkamera ist.

## Claims

1. A method for determining the quality of substitute fuel particles, comprising the following steps:
a. irradiating substitute fuel particles with at least one light source (2),
b. projecting the light reflected and/or transmitted by the substitute fuel particles onto at least one photosensor (3),
c. detecting at least one light spectrum of a substitute fuel particle from the reflected and/or transmitted light by means of the at least one photosensor (3) and transferring the detected light spectrum to an evaluation unit (4) connected to the photosensor (3),
d. comparing, in at least some areas, the detected light spectrum to reference light spectra of reference substitute fuel particles of known materials, determining a difference between the reference light spectra and the detected light spectrum, and associating, by means of the evaluation unit (4), the material of the reference substitute fuel particles whose reference light spectrum has the smallest difference to the detected light spectrum to the detected light spectrum,
**characterized by** the step of
e. determining at least one quantitative quality parameter of the substitute fuel particle whose light spectrum was detected by evaluating, at least in some areas, the detected light spectrum by means of a material-specific correlation function, determined through a regression method, between light spectra data of substitute fuel particles and reference-analytically determined quantitative quality parameters of substitute fuel particles by means of the evaluation unit (4).

2. The method according to claim 1, **characterized in that** the correlation function correlates light spectra data of substitute fuels with quantitative quality parameters selected from moisture content, chlorine content, lower and higher heating values.

3. The method according to one of the claims 1 or 2, **characterized in that** the correlation function is determined through a regression method from the field of statistical machine learning.

4. The method according to claim 3, **characterized in that** the correlation function is determined through a regression method selected from partial least squares regression, main component regression, multiple linear regression, support vector regression, neural network regression, and deep learning methods.

5. The method according to any one of the claims 1 to 4, **characterized in that** the substitute fuel particles are irradiated by the light source (2) with light in a wavelength range of 300-2500 nm or a subrange thereof.

6. The method according to any one of the claims 1 to 4, **characterized in that** the substitute fuel particles are irradiated by the light source (2) with radiation having a discrete spectral pattern.

7. The method according to any one of the claims 1 to 6, **characterized in that** the photosensor (3) creates a hyperspectral image of the substitute fuel particles.

8. A device (1) for determining the quality of substitute fuel particles having a light source (2), a photosensor (3) and an evaluation unit (4) connected to the photosensor (3), wherein the light source (2) is adapted to irradiate substitute fuel particles, and the photosensor (3) is adapted to project light reflected and/or transmitted by the substitute fuel particles onto the photosensor (3), and the photosensor (3) is adapted to detect at least one light spectrum of substitute fuel particles from the reflected and/or transmitted light and to transfer the determined light spectrum to the evaluation unit (4), wherein the evaluation unit (4) is adapted to compare the detected light spectrum, at least in some areas, to reference light spectra of reference substitute fuel particles of known materials, to determine a difference between the reference light spectra and the detected light spectrum, and to associate the material of the reference substitute fuel particles whose reference light spectrum has the smallest difference to the detected light spectrum to the detected light spectrum,
**characterized in that**
the evaluation unit (4) is adapted to determine at least one quantitative quality parameter of the substitute fuel particles whose light spectrum was determined by evaluating, at least in some areas, the detected light spectrum by means of a material-specific correlation function, determined through a regression method, between light spectra data of substitute fuel particles and reference-analytically determined quantitative quality parameters of substitute fuel particles.

9. The device (1) according to claim 8, **characterized in that** the device (1) comprises an output unit (6) connected to the evaluation unit (4), wherein the output unit (6) is adapted to output at least one of the at least one determined quantitative quality parameter, the assigned material, and the determined light spectrum.

10. The device (1) according to one of the claims 8 or 9, **characterized in that** the device (1) comprises a conveyor unit (5), wherein the conveyor unit (5) is adapted to move substitute fuel particles by the light source (2).

11. The device (1) according to claim 10, **characterized in that** the conveyor unit (5) is connected to the evaluation unit (4), wherein the conveyor unit (5) is adapted to stop when the determined quantitative qualitative parameter exceeds or falls below an alert value.

12. The device (1) according to any one of the claims 8 to 11, **characterized in that** the evaluation unit (4) is connected to a signal unit, wherein the signal unit is adapted to produce a signal when the determined quantitative qualitative parameter exceeds or falls below an alert value.

13. The device (1) according to any one of the claims 8 to 12, **characterized in that** the photosensor (3) is a hyperspectral camera.

## Revendications

1. Procédé de détermination de la qualité de particules de combustible dérivé de déchets, comprenant les étapes suivantes :
a. irradier des particules de combustible dérivé de déchets au moyen d'au moins une source de lumière (2),
b. projeter la lumière réfléchie et/ou transmise par les particules de combustible dérivé de déchets sur au moins un photocapteur (3),
c. déterminer au moins un spectre lumineux d'une particule de combustible dérivé de déchets à partir de la lumière réfléchie et/ou transmise par ledit au moins un photocapteur (3) et transmettre le spectre lumineux détecté à une unité d'évaluation (4) qui est reliée au photocapteur (3), et
d. comparer, au moins par endroits, le spectre lumineux détecté avec des spectres lumineux de référence de particules de combustible dérivé de déchets de référence provenant de matériaux connus, déterminer un écart des spectres lumineux de référence par rapport au spectre lumineux détecté et associer au spectre lumineux détecté effectuée par l'unité d'évaluation (4) le matériau de la particule de combustible dérivé de déchets de référence dont le spectre lumineux de référence présente le plus petit écart par rapport au spectre lumineux détecté,
**caractérisé par** l'étape suivante :
e. déterminer au moins un paramètre de qualité quantitatif de la particule de combustible dérivé de déchets dont le spectre lumineux a été détecté par une évaluation, au moins par endroits, du spectre lumineux détecté à l'aide d'une fonction de corrélation qui est spécifique au matériau déterminé, en utilisant une méthode de régression, entre des données de spectre lumineux de particules de combustible dérivé de déchets et des paramètres de qualité quantitatifs de combustibles dérivés de déchets qui sont déterminés par une analyse de référence effectuée par l'unité d'évaluation (4).

2. Procédé selon la revendication 1, **caractérisé en ce que** la fonction de corrélation établit une corrélation entre des données de spectre lumineux de combustibles dérivés de déchets et des paramètres de qualité quantitatifs qui sont choisis parmi la teneur en humidité, la teneur en chlore, la valeur calorifique et le pouvoir calorifique.

3. Procédé selon l'une quelconque des revendications 1 ou 2, **caractérisé en ce que** la fonction de corrélation est déterminée en utilisant une méthode de régression dans le domaine de l'apprentissage statistique/machine.

4. Procédé selon la revendication 3, **caractérisé en ce que** la fonction de corrélation est déterminée en utilisant une méthode de régression qui est choisie parmi la régression des moindres carrés partiels, la régression des composantes principales, la régression linéaire multiple, la régression à vecteurs de support, la régression de réseau neuronal et les méthodes d'apprentissage en profondeur.

5. Procédé selon l'une quelconque des revendications 1 à 4, **caractérisé en ce que** les particules de combustible dérivé de déchets sont irradiées par la source de lumière (2) au moyen de la lumière dans une plage de longueurs d'onde qui est comprise entre 300 nm et 2 500 nm ou dans une zone partielle de celle-ci.

6. Procédé selon l'une quelconque des revendications 1 à 4, **caractérisé en ce que** les particules de combustible dérivé de déchets sont irradiées par la source de lumière (2) au moyen d'un rayonnement qui présente une caractéristique spectrale discrète.

7. Procédé selon l'une quelconque des revendications 1 à 6, **caractérisé en ce que** le photocapteur (3) réalise une image hyperspectrale de la particule de combustible dérivé de déchets.

8. Dispositif (1) de détermination de la qualité de particules de combustible dérivé de déchets comprenant une source de lumière (2), un photocapteur (3) ainsi qu'une unité d'évaluation (4) qui est reliée au photocapteur (3), la source de lumière (2) étant conçue de manière à irradier des particules de combustible dérivé de déchets et le photocapteur (3) étant agencé de manière projeter sur le photocapteur (3) la lumière réfléchie et/ou transmise par les particules de combustible dérivé de déchets et le photocapteur (3) étant conçu de manière à détecter au moins un spectre lumineux d'une particule de combustible dérivé de déchets à partir de la lumière réfléchie et/ou transmise et à transmettre à l'unité d'évaluation (4) le spectre lumineux détecté, l'unité d'évaluation (4) étant conçue de manière à comparer, au moins par endroits, le spectre lumineux détecté avec des spectres lumineux de référence de particules de combustible dérivé de déchets de référence provenant de matériaux connus, à déterminer un écart des spectres lumineux de référence par rapport au spectre lumineux détecté et à associer au spectre lumineux détecté le matériau de la particule de combustible dérivé de déchets de référence dont le spectre lumineux de référence présente le plus petit écart par rapport au spectre lumineux détecté,
**caractérisé en ce que** l'unité d'évaluation (4) est conçue de manière à déterminer au moins un paramètre de qualité quantitatif de la particule de combustible dérivé de déchets dont le spectre lumineux a été détecté par une évaluation, au moins par endroits, du spectre lumineux détecté à l'aide d'une fonction de corrélation qui est spécifique au matériau déterminé, en utilisant une méthode de régression, entre des données de spectre lumineux de particules de combustible dérivé de déchets et des paramètres de qualité quantitatifs de combustibles dérivés de déchets qui sont déterminés par une analyse de référence.

9. Dispositif (1) selon la revendication 8, **caractérisé en ce que** le dispositif (1) comprend une unité de sortie (6) qui est reliée à l'unité d'évaluation (4), l'unité de sortie (6) étant conçue de manière à sortir au moins un élément parmi ledit au moins un paramètre de qualité quantitatif déterminé, le matériau associé et le spectre lumineux détecté.

10. Dispositif (1) selon l'une quelconque des revendications 8 ou 9, **caractérisé en ce que** le dispositif (1) comprend une unité de transport (5), l'unité de transport (5) étant conçue de manière à faire passer des particules de combustible dérivé de déchets au niveau de la source de lumière (2).

11. Dispositif (1) selon la revendication 10, **caractérisé en ce que** l'unité de transport (5) est reliée à l'unité d'évaluation (4), l'unité de transport (5) étant conçue de manière à s'arrêter si le paramètre de qualité quantitatif déterminé dépasse et/ou reste inférieur à une valeur d'alarme.

12. Dispositif (1) selon l'une quelconque des revendications 8 à 11, **caractérisé en ce que** l'unité d'évaluation (4) est reliée à une unité de signalisation, l'unité de signalisation étant conçue de manière à générer un signal si le paramètre de qualité quantitatif déterminé dépasse et/ou reste inférieur à une valeur d'alarme.

13. Dispositif (1) selon l'une quelconque des revendications 8 à 12, **caractérisé en ce que** le photocapteur (3) est une caméra hyperspectrale.
